(19) Europäisches Patentamt
European Patent Office
Office européen des brevets

(11) Veröffentlichungsnummer : **0 444 293 B1**

(12) # EUROPÄISCHE PATENTSCHRIFT

(45) Veröffentlichungstag der Patentschrift :
**18.11.93 Patentblatt 93/46**

(51) Int. Cl.$^5$ : **C07C 69/96,** C07C 68/06

(21) Anmeldenummer : **90124852.6**

(22) Anmeldetag : **20.12.90**

(54) **Verfahren zur Herstellung von mindestens eine aromatische Estergruppe enthaltenden Kohlensäurediestern.**

(30) Priorität : **02.03.90 DE 4006520**

(43) Veröffentlichungstag der Anmeldung :
**04.09.91 Patentblatt 91/36**

(45) Bekanntmachung des Hinweises auf die Patenterteilung :
**18.11.93 Patentblatt 93/46**

(84) Benannte Vertragsstaaten :
**CH DE FR GB IT LI**

(56) Entgegenhaltungen :
**EP-A- 0 338 760**
**DE-A- 3 445 552**
**DE-A- 3 445 555**

(73) Patentinhaber : **BAYER AG**
**D-51368 Leverkusen (DE)**

(72) Erfinder : **Schön, Norbert, Dr.**
**Wilhelmshofallee 82**
**W-4150 Krefeld (DE)**
Erfinder : **Buysch, Hans-Josef, Dr.**
**Brandenburger Strasse 28**
**W-4150 Krefeld (DE)**
Erfinder : **Ebert, Wolfgang, Dr.**
**Doerperhofstrasse 31**
**W-4150 Krefeld (DE)**

**EP 0 444 293 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft ein Verfahren zur Herstellung von mindestens eine aromatische Estergruppe enthaltenden Kohlensäurediestern aus mindestens eine aliphatische Estergruppe enthaltenden Kohlensäurediestern durch katalysierte Umesterung, wobei als Katalysatoren polymere Hydroxystannoxane eingesetzt werden.

Die Herstellung von aromatischen und aliphatisch-aromatischen Kohlensäureestern durch Umesterung, ausgehend von aliphatischen Kohlensäureestern und Phenolen ist prinzipiell bekannt, Dabei handelt es sich um eine Gleichgewichtsreaktion, wobei die Lage des Gleichgewichts fast vollständig in Richtung der aliphatisch substituierten Kohlensäureester verschoben ist. Daher ist es verhältnismäßig leicht, aus aromatischen Kohlensäureestern und Alkoholen aliphatische Kohlensäureester herzustellen, während die Reaktion im umgekehrten Sinn nur dann gut gelingt, wenn sehr reaktive und selektive Katalysatoren zur Verfügung stehen. Daher hat es nicht an Versuchen gefehlt, geeignete Katalysatoren für den genannten Zweck zu entwickeln. Beispielsweise wurden zur Umesterung von aliphatischen Kohlensäureestern mit Phenolen starke Basen, wie Alkalihydroxid, vorgeschlagen. Diese Katalysatoren sind jedoch nur wenig reaktiv und wenig selektiv, da in einer Nebenreaktion in erheblichem Maße Kohlendioxid unter gleichzeitiger Bildung von Ethern herausgespalten wird.

Die in DE-A 2 528 412 und 2 552 907 zur Herstellung von aromatischen Kohlensäureestern empfohlenen Lewis-Säure-Katalysatoren aus der Gruppe der Metallhalogenide oder entsprechender Acyloxy-, Alkoxy- und Aryloxy-Verbindungen von Al, Ti, U, V, Zn, Fe und Sn sind nur im Falle der Titanverbindungen ausreichend wirksam und selektiv. Die Titankatalysatoren haben aber den Nachteil, daß sie die Endprodukte stark rot bis braun färben, was vor allem bei Produkten, die schwierig durch Destillation und Umkristallisation zu reinigen sind, negativ ins Gewicht fällt.

In DE-A 3 445 552 werden für die genannte Umesterung polymere Zinnverbindungen mit wiederkehrenden Struktureinheiten der Formel

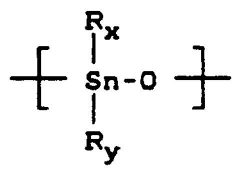

(I)

empfohlen, worin

$R_x$ und $R_y$ jeweils einwertige Kohlenwasserstoffreste bedeuten.

Es wurde nun überraschenderweise gefunden, daß bei der Substitution von Alkylestergruppen durch Arylestergruppen in Kohlensäurediestern durch Umesterung polymere Hydroxystannoxane der nachfolgenden Formel (II) als Katalysatoren erheblich wirksamer sind, als die polymeren Zinnkatalysatoren der obigen Formel (I), wobei gleichzeitig eine sehr hohe Selektivität erreicht wird.

Die Erfindung betrifft demnach ein Verfahren zur Herstellung von mindestens eine aromatische Estergruppe enthaltenden Kohlensäurediestern aus mindestens eine aliphatische Estergruppe enthaltenden Kohlensäurediestern durch katalysierte Umsetzung, daß dadurch gekennzeichnet ist, daß als Katalysator ein polymeres Hydroxystannoxan oder ein Gemisch mehrerer von ihnen mit Monomereinheiten der Formel

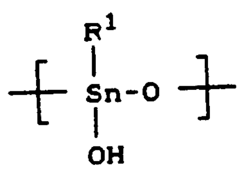

(II),

in der

$R^1$ geradkettiges oder verzweigtes $C_1$-$C_{18}$-Alkyl, $C_3$-$C_8$-Cycloalkyl, $C_6$-$C_{14}$-Aryl, $C_7$-$C_{15}$-Aralkyl oder einen zwei polyhydroxystannoxanketten verbrückenden $C_1$-$C_{18}$-Alkylenrest oder $C_6$-$C_{14}$-Arylenrest bedeutet, in einer Menge von 0,001-20 Gew.-%, bezogen auf den eingesetzten mindestens eine aliphatische Estergruppe enthaltenden Kohlensäurediester, eingesetzt wird.

Geradkettiges oder verzweigtes $C_1$-$C_{18}$-Alkyl ist beispielsweise Methyl, Ethyl, Propyl, Isopropyl, Butyl, Isobutyl, t.-Butyl, die isomeren Pentyle, Hexyle, Octyle (u.a. 2-Ethylhexyl), Decyle, Dodecyle, Hexadecyle und

Octadecyle.

$C_3$-$C_8$-Cycloalkyl ist beispielsweise Cyclopropyl, Cyclopentyl, Methyl-cyclopentyl, Cyclohexyl, Methyl-cyclohexyl, Cycloheptyl und Cyclooctyl.

$C_6$-$C_{14}$-Aryl ist beispielsweise Phenyl, Biphenylyl, Naphthyl, Anthryl.

$C_7$-$C_{15}$-Aralkyl ist beispielsweise Benzyl, Phenylethyl, Phenylpropyl, Phenylbutyl, Naphthyl-methyl, Naphthyl-ethyl, Anthryl-methyl.

$C_1$-$C_{18}$-Alkylenreste, die zwei Polyhydroxystannoxanketten verbrücken, können geradkettig oder verzweigt sein und sind beispielsweise lineare und verzweigte Alkylenreste wie 1,2-Ethylen, 1,2- und 1,3-Propylen, 1,4-Butylen, 1,6-Hexylen etc.

$C_6$-$C_{14}$-Arylenreste, die zwei Polyhydroxystannoxanketten verbrücken, sind beispielsweise 1,2-Phenylen, 1,3-Phenylen, 1,4-Phenylen, 2,2'-Biphenylen, 4,4'-Biphenylen und weitere den obigen aromatischen Resten entsprechende Arylene.

Die in den genannten Substituenten vorhandenen Reste, insbesondere die aromatischen Reste, können ein- bis vierfach durch $C_1$-$C_4$-Alkyl, $C_1$-$C_4$-Alkoxy, $C_1$-$C_4$-Alkylthio, Phenyl, Phenoxy oder Phenylthio substituiert sein.

In bevorzugter Weise werden polymere Hydroxystannoxane der Formel

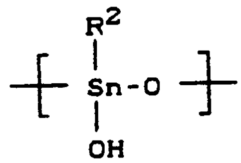

(III)

eingesetzt, worin

$R^2$ geradkettiges oder verzweigtes $C_3$-$C_{12}$-Alkyl, Phenyl oder Benzyl bedeutet.

Selbstverständlich können auch Gemische mehrerer Hydroxystannoxane eingesetzt werden. Ein solches Gemisch kann aus Hydroxystannoxanen bestehen, die unterschiedliche Substituenten aus dem obigen Bedeutungsumfang aufweisen. Ein solches Gemisch kann jedoch auch aus Hydroxystannoxanen bestehen, bei denen unterschiedlich viele wiederkehrende Monomereinheiten der obigen Formel (II) bzw. (III) vorliegen. Die Zahl der wiederkehrenden Monomereinheiten der obigen Formeln kann 3-30, bevorzugt 3-20, betragen.

Besonders bevorzugte polymere Hydroxystannoxane sind solche mit Monomereinheiten der Formel

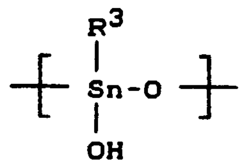

(IV),

in der

$R^3$ geradkettiges oder verzweigtes $C_3$-$C_8$-Alkyl bedeutet.

Beispiele für erfindungsgemäß einsetzbare polymere Hydroxystannoxane sind:
Poly(ethylhydroxystannoxan), Poly(propylhydroxystannoxan), Poly(butylhydroxystannoxan), Poly(octylhydroxystannoxan), Poly(methylhydroxystannoxan), Poly(undecylhydroxystannoxan), Poly(dodecylhydroxystannoxan), Poly(phenylhydroxystannoxan), Poly(4-methylphenyl-hydroxystannoxan) und Poly(4-methoxyphenyl-hydroxystannoxan). Besonders wichtige polymere Hydroxystannoxane sind beispielsweise: Poly (propylhydroxystannoxan), Poly(butylhydroxystannoxan) und Poly(octylhydroxystannoxan).

Die erfindungsgemäß einzusetzenden polymeren Hydroxystannoxane sind beispielsweise gemäß DE-AS 12 27 658 durch alkalische Hydrolyse von Monoorganozinntrihalogeniden zugänglich. Sie liegen, frisch hergestellt, vorzugsweise in trimerer cyclischer Form (Sechsring) vor, gehen aber durch Lagerung, schneller durch thermische Behandlung, in höhermolekulare Spezies über. Hierbei ereignen sich Wasserabspaltung und teilweise auch Vernetzung. Die hauptsächlich vorliegenden cyclischen Oligomeren enthalten keine Endgruppen; bei offenkettigen Spezies liegen Hydroxyl-, Alkoxy-, Halogenendgruppen oder andere dem Fachmann geläufige Endgruppen vor.

Die polymeren Hydroxystannoxane oder ein Gemisch mehrerer von ihnen werden in einer katalytischen Menge eingesetzt, beispielsweise in einer Menge von 0,001-20 Gew.-%, bevorzugt 0,005-5 Gew.-%, besonders bevorzugt 0,005-2 Gew.-%, alles bezogen auf den eingesetzten mindestens eine aliphatische Estergruppe enthaltenden Kohlensäurediester.

Als erfindungsgemäße katalysierte Umesterung ist der Austausch einer oder zweier aliphatischer Estergruppen durch eine oder zwei aromatische Estergruppen zu verstehen. Dies kann beispielsweise die Umsetzung eines aliphatisch-aromatischen Kohlensäurediesters mit einem Phenol zu einem rein aromatischen Kohlensäurediester, gegebenenfalls mit zwei unterschiedlichen aromatischen Estergruppen, sein. Es kann weiterhin die Umsetzung eines rein aliphatischen Kohlensäurediesters mit einem Phenol zu einem aliphatisch-aromatischen oder zu einem rein aromatischen Kohlensäurediester sein. In diesem Fall könnte zunächst, etwa durch Begrenzung der Phenolmenge, eine aliphatische Estergruppe ausgetauscht und danach, gegebenenfalls durch ein unterschiedliches Phenol, die zweite aliphatische Estergruppe ausgetauscht werden, so daß auch hier Kohlensäureester mit zwei verschiedenen aromatischen Estergruppen erhältlich sind.

In beiden genannten Fällen wird der leichter flüchtige aliphatische Esteralkohol destillativ aus dem Umsetzungsgemisch entfernt. Schließlich kann die erfindungsgemäße katalysierte Umesterung auch die Disproportionierung eines gemischten aliphatisch-aromatischen Kohlensäurediesters zu einem rein aromatischen Kohlensäurediester und einem destillativ ebenfalls abtrennbaren rein aliphatischen Kohlensäurediester sein.

Als Phenol zur Umesterung kann auch ein Bisphenol eingesetzt werden.

Für den Fall der Umesterung unter Einsatz eines Phenols kann das Gewichtsverhältnis zwischen dem eingesetzten mindestens eine aliphatische Estergruppe enthaltenden Kohlensäurediester und einem solchen Phenol in weiten Grenzen variiert werden, beispielsweise von 1:99 bis 99:1, bevorzugt 1:9 bis 9:1. Dabei wird im Falle eines großen Überschusses an Phenol hauptsächlich der Diarylkohlensäurediester, im Fall eines großen Überschusses an rein aliphatischem Kohlensäurediester, vorzugsweise der gemischte aliphatisch-aromatische Kohlensäurediester gebildet. Bei Verwendung von Bisphenolen und mindestens 2 Äquivalenten Kohlensäurediester werden Biscarbonate gebildet, die an den Molekülenden noch aliphatische oder aromatische Monoestergruppen aufweisen. Beim Einsatz etwa äquivalenter Mengen Bisphenol und Kohlensäurediester erhält man zunächst Monocarbonate mit einer freien phenolischen Hydroxylgruppe des Bisphenols. Unter verschärften Reaktionsbedingungen entstehen hieraus oligomere oder polymere aromatische Carbonate. Die verschiedenartig substituierten Kohlensäurediester können problemlos, beispielsweise durch Destillation, voneinander getrennt werden.

Erfindungsgemäß eizusetzende mindestens eine aliphatische Estergruppe enthaltende Kohlensäurediester sind solche der Formel

$$R^4O\text{-}CO\text{-}OR^5 \qquad (V),$$

worin

$R^4$ und $R^5$ unabhängig voneinander geradkettiges oder verzweigtes $C_1$-$C_{12}$-Alkyl oder $C_3$-$C_8$-Cycloalkyl bedeuten, wobei weiterhin $R^4$ substituiertes oder nicht substituiertes $C_6$-$C_{12}$-Aryl bedeuten kann.

In bevorzugter Form werden Kohlensäurediester der Formel

$$R^6O\text{-}CO\text{-}OR^7 \qquad (VI)$$

eingesetzt, in der

$R^6$ und $R^7$ unabhänig voneinander geradkettiges oder verzweigtes $C_1$-$C_8$-Alkyl, Cyclopropyl, Cyclopentyl oder Cyclohexyl bedeuten, wobei weiterhin $R^6$ substituiertes oder nicht substituiertes Phenyl bedeuten kann.

Alkyl und Cycloalkyl haben den oben genannten Bedeutungsumfang. $C_6$-$C_{12}$-Aryl ist beispielsweise Phenyl, Biphenylyl oder Naphthyl, bevorzugt Phenyl. Für den Fall einer Substitution von $C_6$-$C_{12}$-Aryl kommen ein oder zwei Substituenten aus der Gruppe $C_1$-$C_4$-Alkyl, $C_1$-$C_4$-Alkoxy, Cyano, Fluor, Chlor, Brom (Chlor ist bevorzugtes Halogen) und Nitro in Betracht.

Wichtige einzusetzende zwei aliphatische Estergruppen enthaltende Kohlensäurediester sind beispielsweise:

Dimethylcarbonat, Diethylcarbonat, Dibutylcarbonat, Diisopropylcarbonat, Dicyclohexylcarbonat, Dioctylcarbonat und besonders bevorzugt Dimethyl- und Diethylcarbonat.

Wichtige einzusetzende aliphatisch-aromatische Kohlensäurediester sind beispielsweise:

Methyl-phenyl-carbonat, Ethyl-phenyl-carbonat, Butyl-phenyl-carbonat, Methyl-kresyl-carbonat und ihre Homologen.

Für den Fall, daß die Umesterung mit Hilfe eines Phenols vorgenommen wird, wird ein solches der Formel

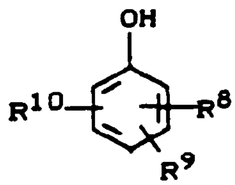

(VII)

eingesetzt, in der

$R^8$ Wasserstoff, $C_1$-$C_4$-Alkyl, $C_2$-$C_4$-Alkenyl, $C_1$-$C_4$-Alkoxy, $C_5$-$C_6$-Cycloalkyl, Phenyl, Fluor, Chlor, Brom, Cyano oder Nitro bedeutet;

$R^9$ für Wasserstoff, $C_1$-$C_4$-Alkyl, $C_2$-$C_4$-Alkenyl, $C_1$-$C_4$-Alkoxy, Fluor, Chlor oder Brom steht und

$R^{10}$ Wasserstoff, $C_1$-$C_4$-Alkyl oder die Gruppe

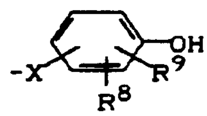

darstellt, in der X eine Einfachbindung, $-CH_2-$, $C_2$-$C_5$-Alkylen, $C_2$-$C_5$-Alkyliden, $C_5$-$C_6$-Cycloalkylen, $C_5$-$C_6$-Cycloalkyliden, Sauerstoff, Schwefel, -CO-, -SO- oder $-SO_2-$ bedeutet, wobei $R^9$ und $R^{10}$ gemeinsam auch einen anellierten Benzolkern bedeuten können.

Alkylengruppen sind über zwei verschiedene C-Atome mit den aromatischen Kernen verbunden, also in 1,2-, 1,3-, 1,4-, 1,5-, 2,3- oder 2,4-Verknüpfung; Alkylidengruppen sind über das gleiche C-Atom mit den aromatischen Kernen verbunden, also in 1,1-, 2,2- oder 3,3-Verknüpfung. Cycloalkylen und Cycloalkyliden können 1 bis 3-fach durch Methyl oder Ethyl substituiert sein.

Als Monophenole werden zur Umesterung in bevorzugter Weise solche der Formel

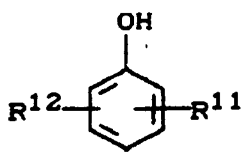

(VIII)

eingesetzt, in der

$R^{11}$ und $R^{12}$ unabhängig voneinander Wasserstoff, $C_1$-$C_4$-Alkyl, $C_5$-$C_6$-Cycloalkyl oder Chlor bedeuten, wobei $R^{11}$ zusätzlich Nitro bedeuten kann.

Als Phenole seien beispielhaft genannt:

Nicht substituiertes Phenol, o-, m- oder p-Kresol, o-, m- oder p-Chlorphenol, o-, m- oder p-Ethylphenol, o-, m- oder p-Propylphenol, o-, m- oder p-Nitrophenol, 2,6-Dimethylphenol, 2,4-Dimethylphenol und 3,4-Dimethylphenol.

Bevorzugte Bisphenole sind solche der Formel

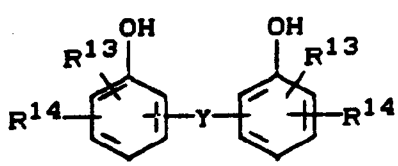

(IX),

in der

$R^{13}$ und $R^{14}$ unabhängig voneinander Wasserstoff, $C_1$-$C_4$-Alkyl, $C_2$-$C_4$-Alkenyl, $C_1$-$C_4$-Alkoxy, $C_5$-$C_6$-Cycloalkyl, Fluor, Chlor oder Brom bedeuten, wobei $R^{13}$ zusätzlich Nitro bedeuten kann, und

Y für eine Einfachbindung, $-CH_2-$, $C_2$-$C_5$-Alkyliden, $C_5$-$C_{10}$-Cycloalkyliden, Schwefel oder $-SO_2-$ steht.

Als Bisphenole werden zur Umesterung in besonders bevorzugter Weise solche eingesetzt, in denen X oder Y und die Hydroxylgruppen in o-,o'-, p-,p'- oder o-,p'-Stellung zueinander stehen.

Ganz besonders bevorzugte Bisphenole sind solche der Formeln

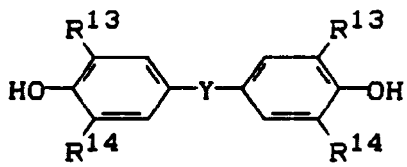

(X)

bzw.

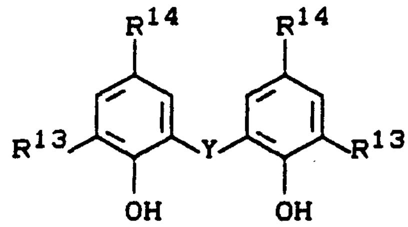

(XI)

bzw.

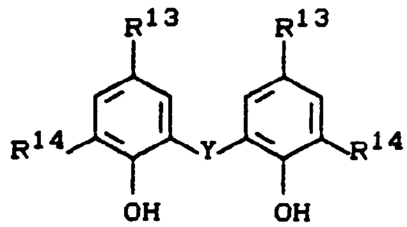

(XII)

in denen $R^{13}$, $R^{14}$ und Y die obengenannte Bedeutung haben.

Als Bisphenole seien beispielsweise genannt:

2,2-Bis(4'-hydroxyphenyl)-propan (= Bisphenol A), Bis(4-hydroxyphenyl)-methan, 1,1-Bis(4'-hydroxyphenyl)-cyclohexan, 2,2-Bis(3',5'-dimethyl-4'-hydroxyphenyl)-propan, 2,2'- und 4,4'-Dihydroxy-biphenyl, Bis(5-methyl-2-hydroxyphenyl)-methan, Bis(3,5-dimethyl-2-hydroxyphenyl)-methan, Bis(3-tert.-butyl-5-methyl-2-hydroxyphenyl)-methan, 1,1-Bis-(4'-hydroxyphenyl-)3,5,5-trimethylcyclohexan. Besonders wichtig unter diesen ist das Bisphenol A.

Erfindungsgemäß erhältliche mindestens eine aromatische Estergruppe enthaltende Kohlensäurediester sind solche der Formel

$$R^{15}O\text{-}CO\text{-}OR^{16} \qquad \text{(XIII)},$$

in der

$R^{15}$ und $R^{16}$ unabhängig voneinander substituiertes oder nicht substituiertes $C_6$-$C_{12}$-Aryl bedeuten kann, das in der oben angegebenen Weise substituiert sein kann, wobei weiterhin $R^{15}$ geradkettiges oder verzweigtes $C_1$-$C_{12}$-Alkyl oder $C_3$-$C_8$-Cycloalkyl bedeuten kann.

Das erfindungsgemäße Verfahren wird bei einer Temperatur von 50-300° C, bevorzugt 100-250° C, durchgeführt. Der Druck ist grundsätzlich nicht kritisch und kann im weiten Bereich von 0,5-50 bar, bevorzugt 1-10 bar liegen.

Erfindungsgemäß kann ohne jedes Lösungsmittel, also in der Schmelze der umzusetzenden Stoffe, gearbeitet werden. Es ist jedoch gleichermaßen möglich, in einem bezüglich der Reaktion inerten Lösungsmittel zu arbeiten. Die Arbeitsweise unter Benutzung eines solchen inerten Lösungsmittels kann beispielsweise dann von Bedeutung sein, wenn der durch die Umesterung entstehende aliphatische Alkohol mit Hilfe eines solchen Lösungsmittels leichter aus den Reaktionsgemisch entfernt werden kann. Lösungsmittel die erfindungsgemäß geeignet sind, sind beispielsweise:

Aromatische (Halogen)Kohlenwasserstoffe, wie Toluol, Xylole, Chlorbenzol, Dichlorbenzole, Trimethylbenzole, Biphenyl, und (cyclo)aliphatische Kohlenwasserstoffe, wie Hexan, Heptan, Isooctan, Cyclohexan, Dekalin, Ligroin, Petrolether, sowie aliphatische und aromatische Nitrile und Ketone, wie Aceton, Acetonitril, Acetylbenzol, Benzonitril und andere.

Eine mögliche Verfahrensweise besteht darin, daß das Umesterungsgemisch in einer Apparatur mit einer ausreichenden Kolonne auf die gewünschte Reaktionstemperatur gebracht wird und der herauszuspaltende aliphatische Alkohol über Kopf abdestilliert wird. Für den Fall einer Disproportionierung, bei der aus einem aliphatischaromatischen Kohlensäurediester der vollständig aromatische Kohlensäurediester und der vollständig aliphatische Kohlensäurediester entstehen, kann der im allgemeinen leichter flüchtige vollständig aliphatische Kohlensäurediester über Kopf abdestilliert werden. Die Abtrennung des leichter flüchtigen Stoffes kann in der

beschriebenen Weise unter Benutzung eines inerten Lösungsmittels oder Gasstromes erfolgen. Weiterhin ist es möglich, einen oder beide Ausgangsstoffe nur zum Teil vorzulegen und nach Anlaufen der Reaktion in die Schmelze oder Lösung des Reaktionsgemisches nachzudosieren.

Die nachfolgenden Beispiele zeigen die besonders gesteigerte katalytische Reaktivität der erfindungsgemäßen polymeren Hydroxystannoxane im Vergleich mit den in der Beschreibungseinleitung genannten Katalysatoren des Standes der Technik.

Beispiele 1-5

Allgemeine Versuchsvorschrift

Zur Bestimmung der katalytischen Wirksamkeit des jeweiligen Katalysators unter gleichen Bedingungen wurde eine Heißextraktionsapparatur, bestehend aus Mehrhalssumpfkolben, einem Extraktionsaufsatz (Rohr mit eingesetzter Extraktionshülse) und Rückflußkühler benutzt. Im Sumpfkolben wurde jeweils ein Gemisch aus 29,5 g (0,25 Mol) Diethylcarbonat und 47,1 g (0,50 Mol) Phenol zum Sieden gebracht, so daß das leichter flüchtige Diethylcarbonat in den Extraktionsaufsatz destillierte und die mit jeweils 10 g Molekularsieb (Baylith TE 144 der Fa. Bayer) gefüllte Extraktionshülse gleichmäßig durchströmte. Durch Zugabe des zu untersuchenden Katalysators (1 mval) in den Sumpfkolben, wurde die Reaktion in Gang gebracht (Zeit t = 0), dabei das gebildete Ethanol aus dem Reaktionsgemisch mit Hilfe von Diethylcarbonat herausgeschleppt und am Molekularsieb selektiv und dauerhaft gebunden. Durch Bestimmung der Produktbildung (Ethylphenylcarbonat und Diphenylcarbonat) in Abhängikeit von der Reaktionszeit mit Hilfe der Gaschromatographie (GC) wurden die Reaktionsgeschwindigkeiten ermittelt.

<u>Tabelle 1</u> (Beispiele 1 bis 5)

| Beispiel | Katalysator | t [h] | EPC[a)] | DC[b)] | EPC +DC | Verfärbung d. Reakt. gemisches |
|---|---|---|---|---|---|---|
| | | | alles in Flächen-% (GC) | | | |
| 1 (erf.gemäß) | 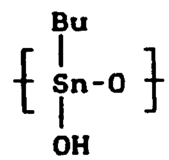 | 1/4 | 4,3 | 0,2 | 4,5 | |
| | | 1/2 | 8,2 | 0,6 | 8,8 | |
| | | 1 | 13,9 | 1,9 | 15,8 | |
| | | 2 | 18,5 | 3,7 | 22,2 | sehr gering |
| 2 (z.Vergl.) | 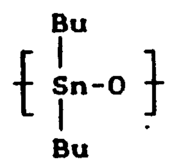 | 1/4 | 0,5 | - | 0,5 | |
| | | 1/2 | 0,9 | - | 0,9 | |
| | | 1 | 1,7 | 0,3 | 2,0 | |
| | | 2 | 3,5 | 0,6 | 4,1 | gering |
| 3 (z.Vergl.) | $Ti(OC_2H_5)_4$ | 1/4 | 1,7 | - | 1,7 | |
| | | 1/2 | 3,4 | 0,2 | 3,6 | |
| | | 1 | 6,5 | 0,7 | 7,2 | |
| | | 2 | 12,2 | 1,9 | 14,2 | orange-rot |

**Tabelle 1** - Fortsetzung -

| Beispiel | Katalysator | t [h] | EPC a) | DC b) | EPC +DC | Verfärbung d. Reakt. gemisches |
|---|---|---|---|---|---|---|
| | | | alles in Flächen-% (GC) | | | |
| 4 (z.Vergl.) | Sn(O-i-$C_8H_{17}$)$_4$ | 1/4 | 0,3 | - | 0,3 | braun |
| | | 1/2 | 0,6 | - | 0,6 | |
| | | 1 | 1,2 | 0,1 | 1,3 | |
| | | 2 | 2,2 | 0,2 | 2,4 | |
| 5 (z.Vergl.) | ohne Kat. | 2 | <0,1 | <0,1 | <0,1 | sehr gering |

a) EPC = Ethylphenylcarbonat  b) DC = Diphenylcarbonat

Aus den Ergebnissen der Beispiele 1-4 erkennt man deutlich die erheblich größere Umesterungsaktivität der erfindungsgemäßen Katalysatoren sowohl in bezug auf Bildung des aliphatisch-aromatischen Ethylphenylcarbonats als auch des diaromatischen Diphenylcarbonats im Vergleich zu den polymeren Zinnkatalysatoren aus Beispiel 2 (entsprechend DE-OS 3 445 552) oder zu den monomeren Titan- und Zinnalkoholaten aus den Beispielen 3 und 4 (entsprechend DE-OS 2 528 412 und DE-OS 2 552 907). Deutlich ist auch die nur geringe Verfärbung im Vergleich mit den Titanestern nach Beispiel 3.

Beispiele 6 bis 9

Nach der in den Beispielen 1 bis 5 beschriebenen Verfahrensweise wurde die Umesterung eines Gemisches aus 35,4 g (0,30 Mol) Diethylcarbonat und 34,2 g (0,15 Mol) Bisphenol A in Abhängigkeit verschiedener Katalysatoren (je 1 mval) untersucht. Mit Hilfe der Gaschromatographie (GC) wurde die Produktbildung (Bisphenol-A-monoethylcarbonat und Bisphenol-A-bisethylcarbonat) in Abhängigkeit von der Reaktionszeit als Maß für die Reaktionsgeschwindigkeit ermittelt.

Tabelle 2 (Beispiele 6 - 9)

| Beispiel | Katalysator | t [h] | BPA-MC[a)] | BPA-BC[b)] | BPA-MC + BPA-BC | Verfärbung |
|---|---|---|---|---|---|---|
| 6 (erf.gemäß) | 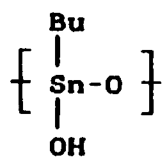 | 1/4<br>1<br>2 | 0,5<br>3,9<br>13,6 | -<br>0,1<br>1,4 | 0,5<br>4,0<br>15,0 | sehr gering |
| 7 (z.Vergl.) | 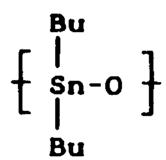 | 1/4<br>1<br>2 | 0,9<br>2,6<br>4,5 | -<br>-<br>0,2 | 0,9<br>2,6<br>4,7 | gering |
| 8 (z.Vergl.) | $Sn(OiC_8H_{17})_4$ | 1/4<br>1<br>2 | 0,1<br>0,2<br>0,5 | -<br>-<br>- | 0,1<br>0,2<br>0,5 | gering |
| 9 (z.Vergl.) | $Ti(OC_{12}H_{25})_4$ | 1/4<br>1<br>2 | Nur minimale Umsetzung, da Ausfällung des Bisphenol-A durch Kat. | | | orange Ausfällung |

a) BPA-MC = Bisphenol-A-monoethylcarbonat

b) BPA-BC = Bisphenol-A-bisethylcarbonat

**Patentansprüche**

1. Verfahren zur Herstellung von mindestens eine aromatische Estergruppe enthaltenden Kohlensäurediestern aus mindestens eine aliphatische Estergruppe enthaltenden Kohlensäurediestern durch katalysierte Umesterung, dadurch gekennzeichnet, daß als Katalysator ein polymeres Hydroxystannoxan oder ein Gemisch mehrerer von ihnen mit Monomereinheiten der Formel

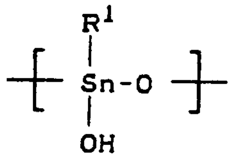
,

in der
$R^1$ geradkettiges oder verzeigtes $C_1$-$C_{18}$-Alkyl, $C_3$-$C_8$-Cycloalkyl, $C_6$-$C_{14}$-Aryl, $C_7$-$C_{15}$-Aralkyl oder einen zwei Polyhydroxystannoxanketten verbrückenden $C_1$-$C_{18}$-Alkylenrest oder $C_6$-$C_{14}$-Arylenrest bedeutet,
in einer Menge von 0,001-20 Gew.-%, bezogen auf den eingesetzten mindestens eine aliphatische Estergruppe enthaltenden Kohlensäurediester, eingesetzt wird.

2. Verfahren nach Anspruch 1, dadurch gekennzeichnet, daß ein polymeres Hydroxystannoxan oder ein Gemisch mehrerer von ihnen mit Monomereinheiten der Formel

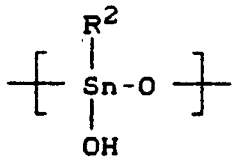

eingesetzt wird, in der
$R^2$ geradkettiges oder verzweigtes $C_3$-$C_{12}$-Alkyl, Phenyl oder Benzyl bedeutet.

3. Verfahren nach Anspruch 2, dadurch gekennzeichnet, daß ein polymeres Hydroxystannoxan oder ein Gemisch mehrerer von ihnen mit Monomereinheiten der Formel

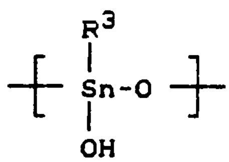

eingesetzt wird, in der
$R^3$ geradkettiges oder verzweigtes $C_3$-$C_8$-Alkyl bedeutet.

4. Verfahren nach Anspruch 1, dadurch gekennzeichnet, daß der Katalysator in einer Menge von 0,005-5 Gew.-%, bevorzugt 0,005-2 Gew.-%, eingesetzt wird.

5. Verfahren nach Anspruch 1, dadurch gekennzeichnet, daß mindestens eine aliphatische Estergruppe enthaltende Kohlensäurediester der Formel

$$R^4O\text{-}CO\text{-}OR^5$$

eingesetzt werden, in der
$R^4$ und $R^5$ unabhängig voneinander geradkettiges oder verzweigtes $C_1$-$C_{12}$-Alkyl oder $C_3$-$C_8$-Cycloalkyl bedeuten, wobei weiterhin $R^4$ substituiertes oder nicht substituiertes $C_6$-$C_{12}$-Aryl bedeuten kann.

6. Verfahren nach Anspruch 5, dadurch gekennzeichnet, daß mindestens eine aliphatische Estergruppe enthaltende Kohlensäurediester der Formel

$$R^6O\text{-}CO\text{-}OR^7$$

eingesetzt werden, in der

$R^6$ und $R^7$ unabhänig voneinander geradkettiges oder verzweigtes $C_1$-$C_8$-Alkyl, Cyclopropyl, Cyclopentyl oder Cyclohexyl bedeuten, wobei weiterhin $R^6$ substituiertes oder nicht substituiertes Phenyl bedeuten kann.

7. Verfahren nach Anspruch 1, dadurch gekennzeichnet, daß zur Umesterung ein Phenol der Formel

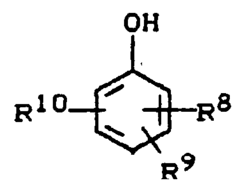

eingesetzt wird, in der

$R^8$ Wasserstoff, $C_1$-$C_4$-Alkyl, $C_2$-$C_4$-Alkenyl, $C_1$-$C_4$-Alkoxy, $C_5$-$C_6$-Cycloalkyl, Phenyl, Fluor, Chlor, Brom, Cyano oder Nitro bedeutet;

$R^9$ für Wasserstoff, $C_1$-$C_4$-Alkyl, $C_2$-$C_4$-Alkenyl, $C_1$-$C_4$-Alkoxy, Fluor, Chlor oder Brom steht und

$R^{10}$ Wasserstoff, $C_1$-$C_4$-Alkyl oder die Gruppe

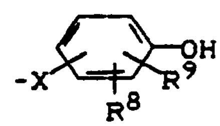

darstellt, in der X eine Einfachbindung, $-CH_2-$, $C_2$-$C_5$-Alkylen, $C_2$-$C_5$-Alkyliden, $C_5$-$C_6$-Cycloalkylen, $C_5$-$C_6$-Cycloalkyliden, Sauerstoff, Schwefel, -CO-, -SO- oder $-SO_2$-bedeutet, wobei $R^9$ und $R^{10}$ gemeinsam auch einen anellierten Benzolkern bedeuten können.

8. Verfahren nach Anspruch 7, dadurch gekennzeichnet, daß ein Phenol der Formel

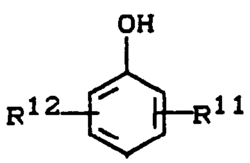

eingesetzt wird, in der

$R^{11}$ und $R^{12}$ unabhängig voneinander Wasserstoff, $C_1$-$C_4$-Alkyl, $C_5$-$C_6$-Cycloalkyl oder Chlor bedeuten, wobei $R^{11}$ zusätzlich Nitro bedeuten kann.

9. Verfahren nach Anspruch 7, dadurch gekennzeichnet, daß ein Bisphenol der Formel

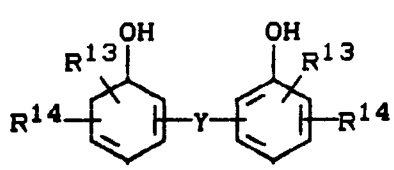

eingesetzt wird, in der

$R^{13}$ und $R^{14}$ unabhängig voneinander Wasserstoff, $C_1$-$C_4$-Alkyl, $C_2$-$C_4$-Alkenyl, $C_1$-$C_4$-Alkoxy, $C_5$-$C_6$-Cycloalkyl, Fluor, Chlor oder Brom bedeuten, wobei $R^{13}$ zusätzlich Nitro bedeuten kann, und

Y für eine Einfachbindung, $-CH_2-$, $C_2$-$C_5$-Alkyliden, $C_5$-$C_{10}$-Cycloalkyliden, Schwefel oder $-SO_2-$ steht.

10. Verfahren nach Anspruch 1, dadurch gekennzeichnet, daß bei einer Temperatur von 50-300° C, bevorzugt 100-250° C gearbeitet wird.

## Claims

1. Process for the preparation of carbonic acid diesters containing at least one aromatic ester group from carbonic acid diesters containing at least one aliphatic ester group by catalysed trans-esterification, characterized in that a polymeric hydroxystannoxane, or a mixture of several of these, containing monomer units of the formula

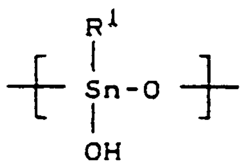
,

in which

$R^1$ denotes straight-chain or branched $C_1$-$C_{18}$-alkyl, $C_3$-$C_8$-cycloalkyl, $C_6$-$C_{14}$-aryl, $C_7$-$C_{15}$-aralkyl or a $C_1$-$C_{18}$-alkylene radical or $C_6$-$C_{14}$-arylene radical bridging two polyhydroxystannoxane chains,

is employed as the catalyst in an amount of 0.001-20% by weight, based on the carbonic acid diester employed which contains at least one aliphatic ester group.

2. Process according to Claim 1, characterized in that a polymeric hydroxystannoxane, or a mixture of several of these, containing monomer units of the formula

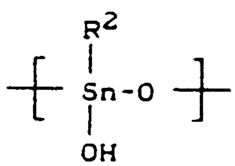

in which

$R^2$ denotes straight-chain or branched $C_3$-$C_{12}$-alkyl, phenyl or benzyl,

is employed.

3. Process according to Claim 2, characterized in that a polymeric hydroxystannoxane, or a mixture of several of these, containing monomer units of the formula

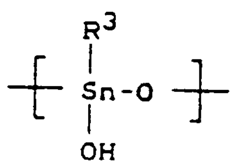

in which

$R^3$ denotes straight-chain or branched $C_3$-$C_8$-alkyl, is employed.

4. Process according to Claim 1, characterized in that the catalyst is employed in an amount of 0.005-5% by weight, preferably 0.005-2% by weight.

5. Process according to Claim 1, characterized in that carbonic acid diesters containing at least one aliphatic

ester group, of the formula

$$R^4O\text{-}CO\text{-}OR^5$$

in which

$R^3$ and $R^5$ independently of one another denote straightchain or branched $C_1$-$C_{12}$-alkyl or $C_3$-$C_8$-cycloalkyl, and wherein $R^4$ can furthermore denote substituted or unsubstituted $C_6$-$C_{12}$-aryl,

are employed.

6. Process according to Claim 5, characterized in that carbonic acid diesters containing at least one aliphatic ester group, of the formula

$$R^6O\text{-}CO\text{-}OR^7$$

in which

$R^6$ and $R^7$ independently of one another denote straight-chain or branched $C_1$-$C_8$-alkyl, cyclopropyl, cyclopentyl or cyclohexyl, and wherein $R^6$ can furthermore denote substituted or unsubstituted phenyl,

are employed.

7. Process according to Claim 1, characterized in that a phenol of the formula

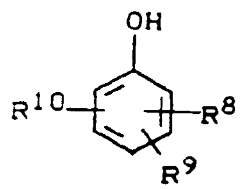

in which

$R^8$ denotes hydrogen, $C_1$-$C_4$-alkyl, $C_2$-$C_4$-alkenyl, $C_1$-$C_4$-alkoxy$_1$ $C_5$-$C_5$-cycloalkyl, phenyl, fluorine, chlorine, bromine, cyano or nitro;

$R^9$ represents hydrogen, $C_1$-$C_4$-alkyl, $C_2$-$C_4$-alkenyl, $C_1$-$C_4$-alkoxy, fluorine, chlorine or bromine and

$R^{10}$ represents hydrogen, $C_1$-$C_4$-alkyl or the group

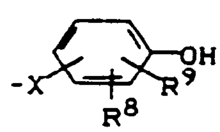

in which X denotes a single bond, $-CH_2-$, $C_2$-$C_5$-alkylene, $C_2$-$C_5$-alkylidene, $C_5$-$C_6$-cycloalkylene, $C_5$-$C_6$-cycloalkylidene, oxygen, sulphur, -CO-, -SO- or $-SO_2-$,

and wherein $R^9$ and $R^{10}$ together can also denote a fused-on benzene nucleus,

is employed for the transesterification.

8. Process according to Claim 7, characterized in that a phenol of the formula

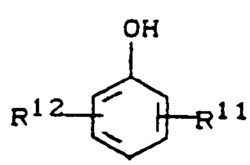

in which

$R^{11}$ and $R^{12}$ independently of one another denote hydrogen, $C_1$-$C_4$-alkyl, $C_5$-$C_6$-cycloalkyl or chlorine, and wherein $R^{11}$ can additionally denote nitro,

is employed.

9. Process according to Claim 7, characterized in that a bisphenol of the formula

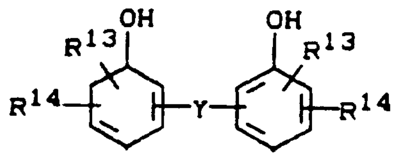

in which

$R^{13}$ and $R^{14}$ independently of one another denote hydrogen, $C_1$-$C_4$-alkyl, $C_2$-$C_4$-alkenyl, $C_1$-$C_4$-alkoxy, $C_5$-$C_6$-cycloalkyl, fluorine, chlorine or bromine, and wherein $R^{13}$ can additionally denote nitro, and

Y represents a single bond, $-CH_2-$, $C_2$-$C_5$-alkylidene, $C_5$-$C_{10}$-cycloalkylidene, sulphur or $-SO_2-$,

is employed.

10. Process according to Claim 1, characterized in that it is carried out at a temperature of 50-300°C, preferably 100-250°C.

## Revendications

1. Procédé de préparation de diesters carboniques contenant au moins un groupe ester aromatique à partir de diesters carboniques contenant au moins un groupe ester aliphatique par transestérification catalysée, caractérisé en ce que l'on utilise en tant que catalyseur un hydroxystannoxane polymère ou un mélange de plusieurs hydroxystannoxanes polymères à motifs monomères de formule

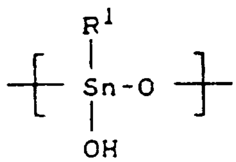

,

dans laquelle

$R^1$ représente un groupe alkyle à chaîne droite ou ramifiée en $C_1$-$C_{18}$, cycloalkyle en $C_3$-$C_8$, aryle en $C_6$-$C_{14}$, aralkyle en $C_7$-$C_{15}$, ou un pont alkylène en $C_1$-$C_{18}$ ou arylène en $C_6$-$C_{14}$ reliant deux chaînes de polyhydroxystannoxane,

en quantité de 0,001 à 20 % du poids du diester carbonique mis en oeuvre qui contient au moins un groupe ester aliphatique.

2. Procédé selon la revendication 1, caractérisé en ce que l'on utilise un hydroxystannoxane polymère ou un mélange de plusieurs hydroxystannoxanes polymères à motifs monomères de formule

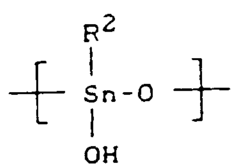

dans laquelle

$R^2$ représente un groupe alkyle à chaîne droite ou ramifiée en $C_3$-$C_{12}$, phényle ou benzyle.

3. Procédé selon la revendication 2, caractérisé en ce que l'on utilise un hydroxystannoxane polymère ou un mélange de plusieurs hydroxystannoxanes polymères à motifs monomères de formule

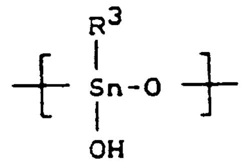

dans laquelle
$R^3$ représente un groupe alkyle à chaîne droite ou ramifiée en $C_3$-$C_8$.

4. Procédé selon la revendication 1, caractérisé en ce que l'on utilise le catalyseur en quantité de 0,005 à 5 % en poids, de préférence de 0,005 à 2 % en poids.

5. Procédé selon la revendication 1, caractérisé en ce que l'on met en oeuvre un diester carbonique contenant au moins un groupe ester aliphatique, de formule

$$R^4O\text{-}CO\text{-}OR^5$$

dans laquelle
$R^4$ et $R^5$ représentent chacun indépendamment l'un de l'autre, un groupe alkyle à chaîne droite ou ramifiée en $C_1$-$C_{12}$ ou cycloalkyle en $C_3$-$C_8$, $R^4$ pouvant en outre représenter un groupe aryle en $C_6$-$C_{12}$ substitué ou non.

6. Procédé selon la revendication 5, caractérisé en ce que l'on met en oeuvre un diester carbonique contenant au moins un groupe ester aliphatique, de formule

$$R^6O\text{-}CO\text{-}OR^7$$

dans laquelle
$R^6$ et $R^7$ représentent chacun, indépendamment l'un de l'autre, un groupe alkyle à chaîne droite ou ramifiée en $C_1$-$C_8$, cyclopropyle, cyclopentyle ou cyclohexyle, $R^6$ pouvant en outre représenter un groupe phényle substitué ou non.

7. Procédé selon la revendication 1, caractérisé en ce que l'on met en oeuvre pour la transestérification un phénol de formule

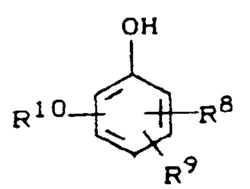

dans laquelle
$R^8$ représente l'hydrogène, un groupe alkyle en $C_1$-$C_4$, alcényle en $C_2$-$C_4$, alcoxy en $C_1$-$C_4$, cycloalkyle en $C_5$-$C_6$, phényle, le fluor, le chlore, le brome, un groupe cyano ou nitro;
$R^9$ représente l'hydrogène, un groupe alkyle en $C_1$-$C_4$, alcényle en $C_2$-$C_4$, alcoxy en $C_1$-$C_4$, le fluor, le chlore ou le brome et
$R^{10}$ représente l'hydrogène, un groupe alkyle en $C_1$-$C_4$ ou le groupe

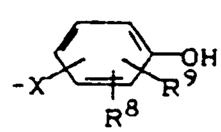

dans lequel X représente un liaison simple, un groupe $-CH_2-$, alkylène en $C_2$-$C_5$, alkylidène en $C_2$-$C_5$, cycloalkylène en $C_5$-$C_6$, cycloalkylidène en $C_5$-$C_6$, l'oxygène, le soufre, -CO-, -SO- ou $-SO_2-$,
$R^9$ et $R^{10}$ pouvant également représenter ensemble un noyau benzénique condensé.

8. Procédé selon la revendication 7, caractérisé en ce que l'on met en oeuvre un phénol de formule

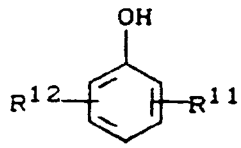

dans laquelle
$R^{11}$ et $R^{12}$ représentent chacun, indépendamment l'un de l'autre, l'hydrogène, un groupe alkyle en $C_1$-$C_4$, cycloalkyle en $C_5$-$C_6$ ou le chlore, $R^{11}$ pouvant en outre représenter un groupe nitro.

**9.** Procédé selon la revendication 7, caractérisé en ce que l'on met en oeuvre un bis-phénol de formule

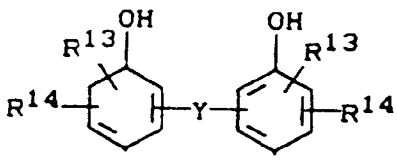

dans laquelle
$R^{13}$ et $R^{14}$ représentent chacun, indépendamment l'un de l'autre, l'hydrogène, un groupe alkyle en $C_1$-$C_4$, alcényle en $C_2$-$C_4$, alcoxy en $C_1$-$C_4$, cycloalkyle en $C_5$-$C_6$, le fluor, le chlore ou le brome, $R^{13}$ pouvant en outre représenter un groupe nitro et
Y représente une liaison simple, $-CH_2-$, un groupe alkylidène en $C_2$-$C_5$, cycloalkylidène en $C_5$-$C_{10}$, le soufre ou $-SO_2-$.

**10.** Procédé selon la revendication 1, caractérisé en ce que l'on opère à une température de 50 à 300°C, de préférence de 100 à 250°C.